(19) Europäisches Patentamt
European Patent Office
Office européen des brevets

(11) Publication number: **0 257 753 A1**

# (12) EUROPEAN PATENT APPLICATION

(21) Application number: **87306036.2**

(22) Date of filing: **08.07.87**

(51) Int. Cl.[4]: **C 07 C 103/44**, C 07 C 103/127, C 07 C 103/375, C 07 D 295/18, C 08 K 5/20, C 08 K 5/34

(30) Priority: **08.07.86 IL 79359**

(43) Date of publication of application: **02.03.88 Bulletin 88/9**

(84) Designated Contracting States: **BE CH DE ES FR GB IT LI NL**

(71) Applicant: **Bromine Compounds Ltd., Makleff House P.O.B. 180, Beer-Sheva 84101 (IL)**

(72) Inventor: **Teuerstein, Avraham, 54 Hadar Street, Omer (IL)**
Inventor: **Ramack, Michael, 8 Shabazi Street, Karkur (IL)**
Inventor: **Zcharia, Reuven, 10 Yiftah Hagil'ad Street, Beer-Sheva (IL)**
Inventor: **Klug, Jacob Thomas, 55/24 Hatzvi Street, Beer-Sheva (IL)**

(74) Representative: **Beresford, Keith Denis Lewis et al, BERESFORD & Co. 2-5 Warwick Court High Holborn, London WC1R 5DJ (GB)**

## (54) Novel derivatives of trihalopivalic acids, method for their preparation and their use.

(57) Trihalopivalic derivatives of the general formula

$$[(CH_2X)_3CCO]_n\text{-}R$$

wherein
X is Cl, Br, or a mixture thereof;
n is 1;
R is $-N<^{R_1}_{R_2}$ in which $R_1$ represents hydrogen or a lower alkyl residue containing 1 to 5 carbon atoms, and $R_2$ represents hydrogen or a lower alkyl residue containing 1 to 5 carbon atoms or a substituted or unsubstituted phenyl residue; or $R_1$ and $R_2$ together form an alicyclic ring;
or wherein
n is 2;
R is $-\overset{R_1}{\overset{|}{N}}-R_3-\overset{R_2}{\overset{|}{N}}-$ in which $R_1$ and $R_2$ are each independently hydrogen or a lower alkyl residue containing 1 to 5 carbon atoms, and $R_3$ represents a phenyl residue or $-(CH_2)_m-$, where m is a number comprised between 1 and 10;
are disclosed, which possess high thermal and UV stability and which can be usefully employed to impart flame-retardant characteristics to synthetic resins.

EP 0 257 753 A1

# NOVEL DERIVATIVES OF TRIHALOPIVALIC ACIDS, METHOD FOR THEIR PREPARATION AND THEIR USE

The present invention relates to novel derivatives of trihalopivalic acids, to methods for their preparation and their use as flame-retardant additives, and to articles obtained therewith.

Tribromopivalic acids, their acid chlorides and a number of their esters are known [Nerdel et al., Chemische Berichte Jahrg., 91 938(1958)]. This article also mentions a number of mono amides of tribromopivalic acid, but no utility is suggested therein for the above compounds. The use of haloalkyl and haloaryl esters of trihalopivalic acids as flame-retardant or plasticizer additives has been disclosed in U.S. Patent No. 3,804,885.

Flame-retardant additives known in the art, and among them the above-mentioned esters, possess different drawbacks such as considerable migration of flame-retardant material in the resin, which causes a degradation of the flame-retardant properties of the material, and low compatibility with many commercially important and useful synthetic plastics. Additives known in the art also often impart to the articles containing them unsatisfactory mechanical properties.

It has now been found, and this is an object of the invention, that alkyl and aryl amides and bis-amides of trihalopivalic acids are

useful as flame-retardant additives and are much more stable than the corresponding esters, provide synthetic resins having improved mechanical properties and thermal stability, and are compatible with a variety of synthetic resins such as polyolefins (e.g., polypropylene), polystyrene and acrylonitrile-butadiene styrene resins, and further present good UV stability.

It has further been found, and this is still another object of the invention, that the compounds of the invention can be usefully employed, in admixture with the synthetic resin, in conventional plastics processing techniques and apparatuses, without the drawbacks inherent to other compounds, such as the corresponding esters.

The flame-retardant trihalopivalic derivatives according to the invention have the general formula

$$[(CH_2X)_3CCO]_n\text{-}R \qquad (I)$$

wherein

X is Cl, Br, or a mixture thereof;

n is 1;

R is $-N\begin{matrix} \diagup R_1 \\ \diagdown R_2 \end{matrix}$ in which $R_1$ represents hydrogen or a lower alkyl

residue containing 1 to 5 carbon atoms, and $R_2$ represents hydrogen or a lower alkyl residue containing 1 to 5 carbon atoms or a substituted or unsubstituted phenyl residue; or
$R_1$ and $R_2$ together form an alicyclic ring;

or wherein
n is 2;

$$\begin{array}{l} \quad\;\; R_1 \quad\;\; R_2 \\ \quad\;\; | \qquad\;\; | \\ R \text{ is } -N-R_3-N- \end{array}$$

in which $R_1$ and $R_2$ are each independently hydrogen or a lower alkyl residue containing 1 to 5 carbon atoms, and $R_3$ represents a phenyl residue or $-(CH_2)_m-$, where m is a number comprised between 1 and 10.

Bis-amides of formula

$$[(CH_2X)_3CCO]_2-R$$

wherein
X is Cl, Br, or a mixture thereof;

$$\begin{array}{l} \quad\;\; R_1 \quad\;\; R_2 \\ \quad\;\; | \qquad\;\; | \\ R \text{ is } -N-R_3-N- \end{array}$$

in which $R_1$ and $R_2$ are each independently hydrogen or a lower alkyl residue containing 1 to 5 carbon atoms, and $R_3$ represents a phenyl residue or $-(CH_2)_m-$, where m is a number comprised between 1 and 10;

are novel compounds, and as such form a part of the present invention.

More particularly, preferred flame-retardant compounds of the invention comprise the trichloro and tribromo pivalic acids amides of tribromoaniline or p-bromoaniline and the trichloro and tribromo pivalic acids amides of m-phenylenediamine, o-phenylenediamine, p-phenylenediamine, 1,2-diaminoethane, 1,2-diaminopropane, 1,3-diaminopropane, 1,4-diaminobutane, 1,6-diaminohexane, 1,4-piperazine, piperidine, pyrrolidine, morpholine, N,N-dimethylethylenediamine, ethanol amine, diethanol amine, aniline, N-methylaniline and diethylamine.

The process for preparing the compounds according to the invention is characterized in that a trihalopivaloyl halide is reacted with the corresponding amine and diamine at a temperature of between 40°C and 150°C.

According to a preferred embodiment of the invention the trihalopivaloyl halide is selected from among trihalopivaloyl chloride and trihalopivaloyl bromide.

According to another preferred embodiment of the invention, the

reaction is carried out in an organic solvent selected from among the chlorinated aromatic or aliphatic hydrocarbons and acetonitrile.

Preferred organic solvents comprise acetonitrile, toluene, chlorobenzene and xylene.

The flame retardant compositions according to the invention are characterized in that they contain as the active ingredient one of the compounds of the invention. The content of the active ingredient in the said composition is preferably of between 2% and 40%, by weight. The content of the said active ingredient varies according to the resin employed and the flame-retardant characteristics that it is desired to impart to it, as it will be apparent to a person skilled in the art. Thus, for instance, the content of active ingredient in styrenic resins will preferably be of between about 4% and about 30%, and in polypropylene of between about 2% and about 8%, for UL 94 V-2 rating, while for different UL 94 V-ratings different amounts of active ingredient will be employed, as it will be apparent to a person skilled in the art. The UL 94 test is a standard test designed to characterize the flame-retardant properties of a given resin. Resins employed for different purposes are required to comply with different V-ratings, according to the UL 94 test, as it is well known in the

art. All V-ratings reported in the following specification have been established according to this standard test.

Preferred flame-retardant composition are those having a melting point equal to or higher than 170°C.

The performance of the synthetic resins employing the compounds of the present invention as flame retardants can be appreciated by testing them for appearance of blooming. When blooming takes place, the concentration of the flame-retardant compound decreases with use and time, and the synthetic resin loses part of its flame-retardant properties, its electric properties (e.g., conductivity) change, and the appearance of the article also changes. It is readily appreciated that it is highly desirable to avoid such occurrence, to maintain a long-term stable quality of the flame-retarded product. It has been found that the compounds of the invention possess a very high resistance to blooming, and therefore resins tested in the accelerated blooming test described below seldom show any significant blooming after an ageing of 1000 hours at 90°C, while under such conditions considerable blooming is observed with commercially available flame-retardant compounds.

Furthermore, commercial plastic processing equipment operates

at temperatures which are dictated by the melting point of the synthetic resin employed. Whenever the flame-resistant composition, which is usually added to the synthetic resin prior to processing, possesses a melting point which is lower than the melting point of the resin or in any case lower than its processing temperature, operating problems occur, both due to the premature melting of the flame-resistant composition, and to the unsatisfactory distribution thereof within the resin, which results from this fact. For instance, polypropylene has a melting point of about 165°C, while styrenic resins have a glass-transition temperature of about 70-80°C and are therefore normally processed at about 170-180°C. It is therefore highly desirable to provide flame-resistant compounds which melt only after melting of the resin is completed or, in any case, at a temperature which is higher than the processing temperature of the resin. This, as stated, is another important advantage of the compounds of the invention.

The characteristics and advantages of the invention will be better understood through the following examples and tests data, which are provided for the purpose of illustration.

## Example 1

Preparation of bis-(tribromopivaloyl) ethylene diamide.

Into a 2 liter, three necked flask, with a mechanical stirrer and reflux condenser, there were placed 1000 ml acetonitrile, 16.5 g of freshly distilled ethylene diamine (0.275 mole), 60 g $Na_2CO_3$ dissolved in 250 ml of water and 200 g of tribromopivaloyl chloride (0.56 mole).

The mixture was stirred under heating at 70°C for 8 hours. Thereafter, the mixture was cooled to 25°C and filtered. The precipitate was thoroughly washed with water and subsequently dried in a vacuum oven at 60°C to a constant weight.

185 g of bis-(tribromopivaloyl) ethylene diamide were obtained (reaction yield: 95%), having a melting point of 238-239°C. The structure of the product was confirmed by nmr, ir and elemental analysis: NMR (∂, $CDCl_3$-TFA) 7.08 (br.s, 2H); 3.76 (s, 12H); 3.56 (m, 4H) [TFA= Trifluroacetic acid; br.s= broad singlet; s= singlet; m=multiplet]. IR(KBr) 3300 $cm^{-1}$ (N-H); 1625$cm^{-1}$ (-CO-NH-). Br content: calcd.: 68.3%, found: 68.0%.

Example 2

Preparation of N-tribromophenyl-tribromopivaloyl amide.
A mixture of 165 g tribromoaniline (0.50 mole), 185 g tribromopivaloyl chloride (0.517 mole), 560 g of $Na_2CO_3$ (0.528 mole), dissolved in 200 ml $H_2O$, was stirred and heated to reflux (about 80°C) for 12 hours, together with 750 ml acetonitrile, in the arrangement of Example 1. After such period, the mixture was cooled to 25°C, filtered and washed several times with water, and subsequently dried in a vacuum oven at 60°C to a constant weight.

296 g of N-tribromophenyl-tribromopivaloyl amide were obtained (reaction yield: 91%), having a melting point of 201-203°C. The structure of the product was confirmed by nmr, ir and elemental analysis. NMR (∂, $CDCL_3$) 7.76 (s, 2H); 3.93 (s, 6H). IR (KBr) 3205 $cm^{-1}$ (N-H); 1645 $cm^{-1}$ (-CO-NH). Br content: calcd.: 73.7%; found: 73.2%.

Example 3

Using the same arrangement as in Example 1, starting from

1,2-diaminopropane (20.5g), the corresponding bis-amide was obtained with a yield of 85% (m.p. 218-219°C).

Example 4

Operating as in Example 3 and starting from 20.5g 1,3-diaminopropane, bis(tribromopivaloyl) propane diamide was obtained with a 90% yield (m.p. 214-215°C).

Example 5

Operating as in Example 3 and starting from 32g 1,6-diaminohexane, bis(tribromopivaloyl) hexanediamide was obtained with a 85% yield (m.p. 194-195°C).

Example 6

Preparation of bis(tribromopivaloyl)piperazine diamide.
3.6 g tribromopivaloyl chloride (0.01 mole), 0.45g piperazine (0.0052 mole), 1.1g pyridine (0.014 mole) and 25 ml methylene chloride were mixed together and heated to reflux (40°C) for 15 hours. The precipitate was filtered off and washed with fresh methylene chloride and then with water. 1.1g of bis(tribromopivaloyl) piperazine diamide were obtained, with a

30% yield (m.p. 228-230°C).

## Example 7

Preparation of N-tribromophenyl-tribromopivaloyl amide. 36g of tribromopivaloyl chloride (0.10 mole) were mixed with 33g of tribromoaniline (0.104 mole) and heated to 120-130°C for 18 hours. The solid was crystallized twice from toluene to give 46g of N-tribromophenyl-tribromopivaloyl amide (70% yield).

## Thermal Gravimetric Analysis

A number of compounds were prepared according to the process of Examples 1 and 2, using the appropriate bases. The compounds so obtained are shown in Table I and their thermal stability was measured on a Mettler TA 3000 System (10°C/min under air), containing a TG-50 Thermobalance unit. The results of these test are also shown in Table I, R and n referring to the above formula I.

Table I: Thermal Gravimetric Analysis (TGA) of some compounds of formula $[(CH_2Br)_3CCONH]_nR$

| | | | % weight loss at T(°C) | | | |
|---|---|---|---|---|---|---|
| Cpd. | R | n | 1% | 2% | 5% | 10% |
| 1 | $Br_3$-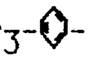- | 1 | 236 | 247 | 263 | 272 |
| 2 | -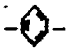- | 2 | 256 | 262 | 269 | 273 |
| 3 | $-(CH_2)_2-$ | 2 | 258 | 260 | 263 | 270 |
| 4 | $-CH_2-CH(CH_3)-$ | 2 | 226 | 235 | 242 | 248 |
| 5 | $-(CH_2)_3-$ | 2 | 244 | 247 | 252 | 255 |
| 6 | $-(CH_2)_6-$ | 2 | 234 | 238 | 244 | 250 |

Blooming Test

The different synthetic resins containing flame-retardant compounds were tested in order to determine their behaviour in the accelerated blooming test. In this test, the synthetic material is kept at 90°C for 1000 hours. Blooming is qualitatively determined by the appearance of a powder on the outer surface of the specimen, due to the migration of the flame-retardant material from the inner sections of the speciment toward the surface. Existence of blooming can be easily confirmed by scratching the surface of the article with a sharp, flexible instrument, and by analysing the powder so removed from the surface.

The specimens tested were prepared using as the synthetic resin Solvay Eltex P HV001P (commercially available polypropylene) containing 0.5% carbon black, a halogenated flame retardant compound and antimony oxide. The Flame-retardant compounds employed were compounds 3 and 4 of Table I, and other known flame-retardant compounds, as detailed in Table II. The results of these tests are shown in Table II, which also reports results for control specimens in which flame-retardant compounds comparable to those of the invention were employed...

Table II: Accelerated Blooming Test

| | Compound Tested | | | | | |
|---|---|---|---|---|---|---|
| | 3 | 4 | A | B | C | D |
| Solvay Eltex P HV001P (%) | 90.5 | 90.4 | 90.5 | 90.7 | 91.0 | 91.3 |
| Flame Retardant (%) | 5.9 | 6.0 | 5.7 | 5.7 | 5.4 | 5.1 |
| Antimony III Oxide (%) | 3.6 | 3.6 | 3.6 | 3.6 | 3.6 | 3.6 |
| Bromine Content (%) | 4 | 4 | 4 | 4 | 4 | 4 |
| Blooming | NO | NO | YES | YES | SLIGHT | YES |
| UL 94 rating, 3.2 mm | V-2 | V-2 | V-2 | V-2 | V-2 | V-2 |

A- Tetrabromobisphenol A-bis(2,3-dibromopropyl ether) (PE 68)

B- $(CH_2Br)_2C[CH_2O\overset{O}{\overset{\|}{C}}-C(CH_2Br)_3]_2$

C- 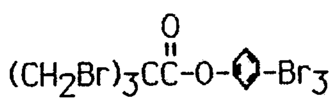

D- 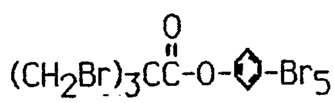

## UV Stability Test

The stability of High Impact Polystyrene (HIPS) containing two different flame-retardant compositions was tested by accelerated UV exposure. White specimens, 127 mm x 12.7 mm x 1.6 mm, were exposed in a Q-Panel Q-U-V Accelerated Weathering Tester. Exposure was for up to 250 hours in the radiation mode. The color change with respect to original color was measured using a Techno-Instruments SCM-90 SpectroColorMeter (CIE 1976 $L^*a^*b^*$ system of measurement). The change of color after 250 hours provides a measure of the stability of the specimen to exposure to UV radiation and is reported in Table III for two compositions: 1) Decabromodiphenyl ether (Deca); and 2) Compound 3 of Table I.

Table III

| Content (wt %) | Deca | Cpd 3 (Table I) |
| --- | --- | --- |
| I.P.E. Galirene HT 88-5 (HIPS) | 76.6 | 73.0 |
| Flame Retardant | 16.9 | 20.5 |
| Antimony III Oxide | 5.1 | 5.1 |
| Irganox PS 800 | 0.5 | 0.5 |
| Irganox 1076 | 0.3 | 0.3 |
| Tinuvin P | 0.3 | 0.3 |
| Irgawax 280 | 0.3 | 0.3 |
| UL 94 rating, 1.6 mm | V-0 | V-0 |
| Color Change (DE) | 42.2 | 26.5 |

From the above data it can be seen that the change in color obtained with the amide of the invention is appreciably lower than that which occurs with the commercially employed Deca flame-retardant, that is, its UV stability is appreciably higher.

The above examples and results have been given for the purpose of illustration, and are not intended to be limitative. Several different embodiments of the invention are possible, as will be apparent to the person skilled in the art. For instance, different solvents and bases can be employed in the synthesis of the compounds, different polymer additives can be used instead of or together with antimony oxide, etc., without exceeding the scope of the invention.

CLAIMS

1. Flame-retardant trihalopivalic derivatives having the general formula

$$[(CH_2X)_3CCO]_n-R$$

wherein

X is Cl, Br, or a mixture thereof, and, when n is 1,

$R_1$
/
R is -N in which $R_1$ represents hydrogen or a lower alkyl
\
$R_2$

residue containing 1 to 5 carbon atoms, and $R_2$ represents hydrogen or a lower alkyl residue containing 1 to 5 carbon atoms or a substituted or unsubstituted phenyl residue; or

$R_1$ and $R_2$ together form an alicyclic ring;

or, when n is 2

$R_1$ $R_2$
| |
R is $-N-R_3-N-$ in which $R_1$ and $R_2$ are each independently hydrogen or a lower alkyl residue containing 1 to 5 carbon atoms, and $R_3$ represents a phenyl residue or $-(CH_2)_m-$, where m is 1 to 10.

2. A compound according to Claim 1, characterized in that it is selected from among the trichloro and tribromo pivalic acids amides of tribromoaniline, p-bromoaniline, m-phenylenediamine, o-phenylenediamine, p-phenylenediamine, 1,2-diaminoethane, 1,2-diaminopropane, 1,3-diaminopropane, 1,4-diaminobutane, 1,6-diaminohexane, 1,4-piperazine, piperidine, pyrrolidine, morpholine, N,N-dimethylethylenediamine, ethanol amine, diethanol amine, aniline, N-methylaniline and diethylamine.

3. A compound of formula

$$[(CH_2X)_3CCO]_2-R$$

wherein

X is Cl, Br, or a mixture thereof;

R is $-\overset{R_1}{\overset{|}{N}}-R_3-\overset{R_2}{\overset{|}{N}}-$ in which $R_1$ and $R_2$ are each independently hydrogen or a lower alkyl residue containing 1 to 5 carbon atoms, and $R_3$ represents a phenyl residue or $-(CH_2)_m-$, where m is a number comprised between 1 and 10.

4. A compound according to Claim 3, characterized in that it is a

trichloro or tribromo pivalic acid amide of m-phenylenediamine, o-phenylenediamine, p-phenylenediamine, 1,2-diaminoethane, 1,2-diaminopropane, 1,3-diaminopropane, 1,4-diaminobutane, 1,6-diaminohexane or N,N-dimethylethylenediamine.

5. A process for preparing compound according to Claims 1 to 4, characterized in that a trihalopivaloyl halide is reacted with the corresponding amine or diamine at a temperature of between 40°C and 150°C.

6. A process according to Claim 5, characterized in that the trihalopivaloyl halide is trihalopivaloyl chloride or trihalopivaloyl bromide.

7. A process according to Claim 5 or 6, characterized in that the reaction is carried out in an organic solvent which is a chlorinated aromatic or aliphatic hydrocarbon.

8. A process according to Claim 5 or 6, characterized in that the reaction is carried out in an organic solvent selected from among acetonitrile, toluene, chlorobenzene and xylene.

9. A flame retardant composition characterized in that it contains as the active ingredient a compound according to Claim

1 or 2, alone in admixture with other polymer additives and modifiers.

10. Composition according to claim 9, characterized in that it contains 2% to 40%, by weight, of the active ingredient.

11. Composition according to claim 10, characterized in that it has a melting point equal to or higher than 170°C.

12. Sythetic resins, whenever made flame-retardant by means of a compound according to claim 1 or 2.

13. Synthetic resins according to claim 12, characterized in that they are chosen from among polypropylene, high impact polystyrene and acrylonitrile-butadiene styrene.

0257753

European Patent Office

# EUROPEAN SEARCH REPORT

Application number

EP 87 30 6036

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-4 179 298 (L.E. NELSON et al.) * Claims * | 1,9-13 | C 07 C 103/44<br>C 07 C 103/127<br>C 07 C 103/375<br>C 07 D 295/18<br>C 08 K 5/20<br>C 08 K 5/34 |
| D,Y | US-A-3 804 885 (C.E. REINEKE et al.) * Claims * | 1,9-13 | |
| P,X | DE-A-3 540 360 (BAYER) * Claims; page 18 * | 1,5 | |
| | | | TECHNICAL FIELDS SEARCHED (Int. Cl.4) |
| | | | C 07 C 103/00<br>C 07 D 295/00<br>C 08 K 5/00 |

The present search report has been drawn up for all claims

| Place of search | Date of completion of the search | Examiner |
|---|---|---|
| THE HAGUE | 19-10-1987 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82